(19) Europäisches Patentamt / European Patent Office / Office européen des brevets

(11) **EP 4 010 764 B1**

(12) **EUROPEAN PATENT SPECIFICATION**

(45) Date of publication and mention
of the grant of the patent:
**19.07.2023   Bulletin 2023/29**

(21) Application number: **20743719.5**

(22) Date of filing: **28.07.2020**

(51) International Patent Classification (IPC):
***G05B 17/02*** *(2006.01)*

(52) Cooperative Patent Classification (CPC):
**G05B 17/02**

(86) International application number:
**PCT/EP2020/071195**

(87) International publication number:
**WO 2021/023566 (11.02.2021 Gazette 2021/06)**

(54) **METHOD OF PRODUCING A CHEMICAL PRODUCT USING A REGRESSION MODEL**

VERFAHREN ZUR HERSTELLUNG EINES CHEMISCHEN PRODUKTS UNTER VERWENDUNG
EINES REGRESSIONSMODELLS

PROCÉDÉ DE PRODUCTION D'UN PRODUIT CHIMIQUE À L'AIDE D'UN MODÈLE DE
RÉGRESSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.08.2019   EP 19190049**

(43) Date of publication of application:
**15.06.2022   Bulletin 2022/24**

(73) Proprietor: **Covestro Intellectual Property GmbH
& Co. KG
51373 Leverkusen (DE)**

(72) Inventors:
• **TABBI, Giuseppe Teodoro Maria
40593 Düsseldorf (DE)**
• **NGO, Tan-Vinh
40625 Düsseldorf (DE)**

(74) Representative: **Levpat
c/o Covestro AG
Gebäude K12
51365 Leverkusen (DE)**

(56) References cited:
**WO-A2-2004/040283     US-A1- 2005 187 445**

**Description**

The present invention relates to a method of producing a chemical product from a reaction mixture. In the method a combined transformed regression model is used. This allows for adjustment of chemical or process parameters in response to local climate parameters such as humidity, temperature or air pressure. The invention is also directed towards a system configured for producing a chemical product and a computing platform configured for producing a chemical product.

In the production of polyurethane foams external, climatic factors may lead to variations in the properties of the final product, for example in regards to the raw density and compression deflection. There is often little choice but to dispose of off-specification material if the process is run throughout a longer period of time where the weather conditions may change.

Before the advent of regression models use was made of heuristic rules, based on the experience of shop personnel. Such individual experience may, however, prove to be too inaccurate to account for many simultaneously changing climate factors and their effect on reaction mixture formulations and process parameters.

The publication "Flexible Polyurethane Slabstock Foam: The Influence of Formulation, Climatic Conditions and Storage Conditions on Foam Properties" by R. Schiffauer and C. den Heijer, Journal of Cellular Plastics Jan/Feb 1983, 61 - 64, reports that:

"The following climatic variables were found to have a significant influence on foam properties: atmospheric pressure during production and absolute humidity during production. The absolute humidity during storage probably also has a significant influence, but owing to the high intercorrelation with absolute humidity during production this effect could not be estimated separately. For compression set, cell count and air flow no significant effects of the climatic variables were found.

The effects of the two dominant climatic variables on the remaining foam properties were: Higher (lower) atmospheric pressures resulted in higher (lower) densities; higher (lower) hardnesses; lower (higher) elongations at break; lower (higher) tensile strengths.

Higher (lower) absolute humidities resulted in no change in densities; lower (higher) hardnesses; higher (lower) elongations at break; higher (lower) tensile strengths.

The similarity of the coefficients for the different grades justified the construction of a combined model using both formulation and climatic variables. Thus, the most important foam properties can be calculated by:

$$\ln(\text{property}) = C_0 + C_1 \ln(\text{water}) + C_2 \ln(\text{blow}) + C_3 \ln(\text{TDI index}) + C_4 \ln(\text{atmospheric pressure}) + C_5 \ln(\text{absolute humidity})".$$

The publication "Mathematical Property Prediction Models for Flexible Polyurethane Foams

- A Comparison between Conventional Slabstock, High Resilience Slabstock and High Resilience Molded Foams" by R. Schiffauer, Journal of Cellular Plastics 1996, 32, pages 318 - 354" reports on a set of foams prepared by varying a range of formulation parameters and that:

"The data were mathematically analyzed by multiple linear regression techniques, using a variety of transformations of the original variables, resulting in equations correlating properties with predicting variables. As a first approach, all data were regressed in their original, linear format. In most cases, however, better fits could be obtained by transformations of either the predicting variables or the resulting properties or both from a linear into a logarithmic format. Further improvements in the resulting equations could be realized by using interaction terms between some predicting properties. When laboratory and production data were analyzed together, a so-called "dummy variable" was assigned (zero for laboratory data and 1 for production results), in order to compute the effect of scaling up on the various properties."

The publication "Innovative Produktionsoptimierung für die Blockschaum-Fertigung" (innovative production optimization for flexible foam production) by Hubert Ehbing, Karl-Heinz Dörner, Hans-Friedrich Walter, Bolko Raffel and R. Näscher in the conference proceedings "PUR 2002 - Automobil - Comfort - Struktur - Kühlen - Bauen", October 2002, pages 192 - 144, discusses a regression model to address the so-called summer/winter effect in the production of flexible foams. The regression model is:

$$\ln(y) = a_0 + a_1 \ln(x_1) + a_2 \ln(x_2) + a_3 \ln(x_3) + \sum_{i=4}^{j} a_i x_i$$

with y = compression hardness; $x_1$ = NCO index; $x_2$ = water; $x_3$ = absolute humidity; $x_4$ = T65 content; $x_5$ = nozzle pressure; $x_6$ = block height; $x_7$ = belt speed.

The above mentioned publications fail to address 4 points: firstly, there is no data cleansing procedure and preprocessing before applying regression models; secondly, the involvement of higher order regressors in the regression model is lacking or missing; thirdly, the possibility of automatically and symbolically solving for generating from the regression models a formula for each recipe component influenced by the climate condition is lacking or missing and fourthly, the deployment of these formulas in a user interface or embedded in an FPGA of the production plant is lacking or missing. US 2005/187445 A1 discloses:A method of producing a chemical product from a reaction mixture containing at least two components.

Without the first two points the influence of climate conditions may have a defective impact on the formulation correction in the production plant. The last two points are beneficial for either giving the plant technician a suggestion to correct the formulation or for performing the correction automatically.

The present invention has the object of at least partially overcoming a drawback in the art. In particular the invention has the object of providing for the production of a chemical product from a reaction mixture where the influence atmospheric parameters on the final product can be compensated by changing the composition of the reaction mixture in a simplified way. This object is achieved by a method according to claim 1, a system according to claim 13 and a computing platform according to claim 14. Advantageous embodiments are the subject of the dependent claims. They may be combined freely unless the context clearly indicates otherwise. It is also within the scope of the present invention that the subject-matter of embodiments described in connection with the method, particularly the in the dependent method claims, may also be applied to the system and computing platform according to the invention. This extends to combinations of embodiments as well.

Accordingly, the method of producing a chemical product from a reaction mixture containing at least two components comprises:

I) providing a data set comprising a plurality of production instances, each production instance comprising data on (a) the composition of the reaction mixture and/or process conditions, (b) at least one climate parameter at the production site of the product, and (c) at least one target physical property of the product;

II) generating a regression model for each target physical property of the product using the data set, wherein in the regression model a respective target physical property is the dependent variable and at least part of the other data in the data set, excluding any remaining target physical properties, are independent variables;

III) transforming each regression model to obtain a transformed regression model where a) the composition of the reaction mixture and/or b) the process conditions of a reaction depends on at least one climate parameter at the production site of the product and each target physical property is assigned a desired value;

IV) combining the transformed regression models to obtain a combined regression model;

V) measuring, at the production site, the at least one climate parameter used in the combined regression model;

VI) calculating a) the composition of the reaction mixture and/or b) the process conditions for the reaction, using the combined regression model and the at least one measured climate parameter;

VII) composing the reaction mixture and/or process conditions according the calculation; and

VIII) reacting the reaction mixture to obtain the chemical product.

The method according to the invention may, at least partially, be a computer-implemented method. Preferably, steps I to VII are computer-implemented steps.

The data set in step I) of the method comprises (represents) a plurality of production instances and may be obtained from data collected as routine process and product monitoring. A production instance may be a single batch or a single campaign in the production of a chemical product. In the case of a continuous production process a production instance may be viewed as a time span in the continuous process. In general, the quality of the data set will improve with greater detail in regards of the production instances.

Besides data on the composition of the reaction mixture and/or the process conditions applicable for the respective production run the production instance comprises data on at least one (preferably two or more) climate parameter at the production site of the product during the respective production run. As this climate data is attributed to the production instance, it may also be described as the respective climate parameters such as air pressure, etc. recorded during the production of the product in the individual instance. In view of the climate parameter(s) recorded, the production of the chemical product is preferably a process where there is at least partially contact of the reaction mixture or one or more of the components of the reaction mixture with the atmosphere.

Lastly, the production instance comprises data on at least one (preferably two or more) target physical property of the product of the respective production run. Such a target physical property in the production instances and therefore in the data set can be viewed as a property which is part of the specifications of the product, which has been determined by measurement for populating the data set and which will be a target to achieve in future production of the chemical product.

In step II), using the data set, a regression model is generated for each target physical property of the product. The target physical property is therefore an output and other data in the data set are inputs. If there are other target physical properties in the data set, they are disregarded. Examples for regression models include least squares sum and maximum likelihood.

In step III) each regression model obtained in step II) is respectively transformed into a model where at least one climate parameter at the production site of the product is an input. It goes without saying that this at least one climate parameter was also included in the data set as described in step I). Furthermore, each target physical property is assigned a value, thereby reflecting the desired outcome of the chemical reaction. It also goes without saying that the target physical property or properties was included in the data set as described in step I). The output of each transformed model is the composition of the reaction mixture and/or the process conditions of a reaction necessary to achieve the target physical properties. In summary, step III) transforms $n$ regression models into $n$ transformed regression models.

Furthermore, the involvement of higher order regressors with interaction can also improve the fit of a regression model. Such a regression model may have the following form:

$$y = \beta_0 + \beta_1 x_1 + \beta_{12} x_1 * x_2 + \beta_2 x_2^2 + \cdots + \beta_m x_i^m + \varepsilon$$

With the fitted regression models for each property $y_i = f(x_j)$ a symbolic solver can be applied in order to receive formulas for correction the formulation parameters $x_1$ and $x_2$.

It is advantageous to use data cleansing and preprocessing techniques to enhance the data quality of the historical data and to improve the fit of the regression model(s). The data cleansing techniques are preferably focused to find outliers and impute them either with a default value or with a statistical descriptor such as the mean and the median of the corresponding parameter.

Hence, in one embodiment the data set is processed prior to step I) using a statistical method to remove outliers. Examples for suitable statistical methods include variance analysis, mutual information and Z-score. Outliers can be removed on the basis of values such as medians or expected values derived from the aforementioned methods. The dimensions of the feature space created by recipe details, atmospheric conditions, process details and properties of the final product may also be reduced to the essential features. The statistical analysis also permits to identify common features or clusters in the data, for example by using scatter plots or principal component methods.

In another embodiment step II) comprises:

splitting the data set into a training data set and a testing data set,

generating each regression model using the training data set, and

validating the regression model against the testing data set.

Candidates for regression models which are validated against the testing data set may be cross-validated according to criteria such as the coefficient of determination $R^2$, the mean absolute error MAE or the root mean square error RMSE. The regression model for each target physical property with the highest $R^2$ and the lowest MAE and RMSE values can then be selected.

In another embodiment the data set is grouped into clusters and each cluster is split into a training data set and a testing data set for generating the regression models.

In another embodiment at least one of steps I) to VI) comprises providing an interaction with a user via a graphical user interface. For example, the calculated formulas for the formulation parameters can be integrated in a user interface, where the plant technician inserts its standard formulation. In the same time the climate condition is measured by sensors implemented in the production plant and foam storage area. The measured information with the standard formulation of the plant technician are consumed by the formulas, which then calculates the climate corrected formulation. The climate corrected formulation is visualized in the user interface, where the plant technician can manually correct the set points.

In another scenario the calculated formulas are implemented in a FPGA, which controls the set points of the machinery. The plant technician inserts its standard formulation in a user interface. The inserted formulation with the measured climate information of the above mentioned sensors are consumed by the FPGA. The FPGA can now change the set points of the machinery without the interaction with the plant technician.

In another embodiment steps V) to VIII) occur in 60 seconds or less.

In another embodiment at least two target physical properties are considered.

In another embodiment at least two climate parameters are considered.

In another embodiment the at least one climate parameter at the production site is the air pressure, the air temperature, the air's relative humidity or a combination thereof.

In another embodiment the at least one target physical property is the raw density according to DIN EN ISO 845 and/or the compression load deflection at 40% compression according to EN ISO 3386.

In another embodiment the chemical product is a polyurethane foam and the reaction mixture comprises a polyisocyanate, a polyisocyanate-reactive compound and a blowing agent. Examples for suitable polyisocyanates include TDI, MDI, H12-MDI, XDI, HDI, IPDI and their derivatives such as oligomers, NCO-terminated prepolymers, biurets, uretdiones, allophanates and isocyanurates. Examples for suitable polyols include polyester polyols, polyester polyols, polycarbonate polyols, polyetherester polyols and polyestercarbonate polyols. Blowing agents can be physical blowing agents such as cyclopentane or chemical blowing agents such as water or formic acid.

In another embodiment composing the reaction mixture in step VII) comprises, starting from a pre-defined standard reaction mixture composition:

(a) increasing or decreasing a molar ratio of isocyanate groups to isocyanate-reactive groups;

(b) increasing or decreasing an amount of blowing agent;

(c) increasing or decreasing relative amounts of physical and chemical blowing agents with respect to each other; or

(d) a combination thereof.

In another embodiment the method further comprises:

IX) measuring, at the production site, the at least one climate parameter used in the combined regression model after step VIII has begun;

X) calculating the composition of the reaction mixture or process conditions of the reaction using the combined regression model and the at least one measured climate parameter in step IX;

XI) adjusting, or deciding not to adjust, the reaction mixture or process conditions according to the calculation in step X; and

XII) reacting the reaction mixture to obtain the chemical product after making the adjustment or the decision not to adjust, the reaction mixture or process conditions in step XI.

It is preferred that steps IX to XII are repeated at least once every 60 seconds, at least once every 30 seconds, or continuously.

In another embodiment the method further comprises:

IX') measuring, at the production site, the at least one climate parameter used in the combined regression model after step VIII has begun;

X') determining if the measurement in step IX' is different from a previous measurement of the at least one climate parameter;

XI') if the measurement has been determined to be different, then calculating the composition of the reaction mixture or process conditions of the reaction using the combined regression model and the at least one measured climate parameter in step IX';

XII') adjusting the reaction mixture or process conditions according to the calculation in step XI', if such a calculation is made; and

XIII') reacting the reaction mixture to obtain the chemical product after making the adjustment, the reaction mixture or process conditions in step XII', if a calculation is made in step XI.

It is preferred that steps IX' to XI' are repeated at least once every 60 seconds, at least once every 30 seconds, or continuously.

It is also preferred that at least two climate parameters are measured in steps V and IX'.

The present invention is furthermore directed towards a system configured for producing a chemical product from a reaction mixture containing at least two components, the system comprising:

one or more hardware processors configured by machine-readable instructions to:

provide a data set comprising a plurality of production instances, each production instance comprising data on the composition of the reaction mixture, at least one climate parameter at the production site of the product, and at least one target physical property of the product;

generate a regression model for each target physical property of the product using the data set, wherein in the regression model a respective target physical property is the dependent variable and other data in the data set are independent variables;

transform each regression model to obtain a transformed regression model where the composition of the reaction mixture and/or the process conditions of a reaction depends on at least one climate parameter at the production site of the product and each target physical property is assigned a desired value;

combine the transformed regression models to obtain a combined regression model;

measuring, at the production site, at least one climate parameter used in the combined regression model;

calculate the composition of the reaction mixture and/or the process conditions for the reaction using the combined regression model and the at least one measured climate parameter; and

compose the reaction mixture and/or process conditions according the calculation;

and a reactor for reacting the reaction mixture to obtain the chemical product.

Another aspect of the present invention is a computing platform configured for producing a chemical product from a reaction mixture containing at least two components, the computing platform comprising:

a non-transient computer-readable storage medium having executable instructions embodied thereon; and

one or more hardware processors configured to execute the instructions to:

provide a data set comprising a plurality of production instances, each production instance comprising data on the composition of the reaction mixture, at least one climate parameter at the production site of the product, and at least one target physical property of the product;

generate a regression model for each target physical property of the product using the data set, wherein in the regression model a respective target physical property is the dependent variable and other data in the data set are independent variables;

transform each regression model to obtain a transformed regression model where the composition of the reaction mixture and/or the process conditions of a reaction depends on at least one climate parameter at the production site of the product and each target physical property is assigned a desired value;

combine the transformed regression models to obtain a combined regression model;

measuring, at the production site, at least one climate parameter used in the combined regression model;

calculate the composition of the reaction mixture and/or the process conditions for the reaction using the combined regression model and the at least one measured climate parameter; and

compose the reaction mixture and/or process conditions according the calculation.

Preferably the computing platform is in data communication with a reactor for reacting the reaction mixture to obtain the chemical product.

The present invention will be further described with reference to the following figures without wishing to be limited by them.

FIG. 1 illustrates a system configured for producing a chemical product from a reaction mixture containing at least two components, in accordance with one or more implementations.

FIG. 2 illustrates a method for producing a chemical product from a reaction mixture containing at least two components, in accordance with one or more implementations.

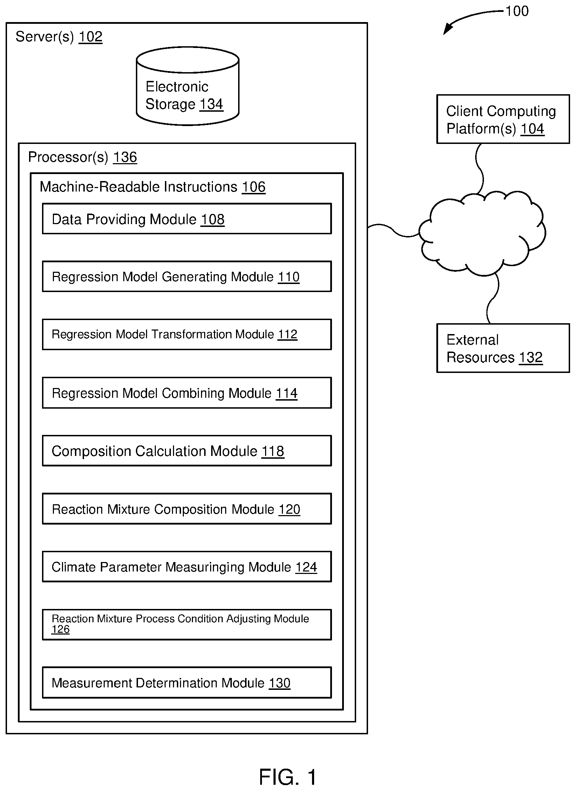

FIG. 1 illustrates a system 100 configured for producing a chemical product from a reaction mixture containing at least two components, in accordance with one or more implementations. In some implementations, system 100 includes one or more servers 102. Server(s) 102 is configured to communicate with one or more client computing platforms 104 according to a client/server architecture and/or other architectures. Client computing platform(s) 104 is configured to communicate with other client computing platforms via server(s) 102 and/or according to a peer-to-peer architecture and/or other architectures. Users may access system 100 via client computing platform(s) 104.

Server(s) 102 is configured by machine-readable instructions 106. Machine-readable instructions 106 include one or more instruction modules. The instruction modules include computer program modules. The instruction modules include one or more of a data providing module 108, a regression model generating module 110, a regression model transformation module 112, a regression model combining module 114, a composition calculation module 118, a reaction mixture composition module 120, a reaction mixture reaction module 122, a climate parameter measuring module 124, a reaction mixture process condition adjusting module 126, a reaction mixture process condition adjusting module 128, a measurement determination module 130, and/or other instruction modules.

Data providing module 108 is configured to provide a data set including a plurality of production instances. By way of non-limiting example, each production instance includes data on the composition of the reaction mixture, at least one climate parameter at the production site of the product, and at least one target physical property of the product. The data set is processed prior to providing the data set using a statistical method to remove outliers. The at least one target physical property is the raw density according to DIN EN ISO 845 and/or the compression load deflection at 40 % compression according to EN ISO 3386.

Regression model generating module 110 is configured to generate a regression model for each target physical property of the product using the data set. Generating the regression model includes splitting the data set into a training data set and a testing data set. Generating the regression model includes generating each regression model using the training data set. Generating the regression model includes validating the regression model against the testing data set. The data set is grouped into clusters and each cluster is split into a training data set and a testing data set for generating the regression models.

In the regression model a respective target physical property is the dependent variable and other data in the data set are independent variables.

Regression model transformation module 112 is configured to transform each regression model to obtain a transformed regression model where the composition of the reaction mixture and/or the process conditions of a reaction depends on at least one climate parameter at the production site of the product and each target physical property is assigned a desired value. By way of non-limiting example, the at least one climate parameter at the production site is the air pressure and/or the air temperature.

Regression model combining module 114 is configured to combine the transformed regression models to obtain a combined regression model.

Composition calculation module 118 is configured to calculate the composition of the reaction mixture and/or the process conditions for the reaction using the combined regression model and the at least one measured climate parameter..

Composition calculation module 118 is alternatively configured to, if the measurement has been determined to be different, calculate the composition of the reaction mixture or process conditions of the reaction using the combined regression model and the at least one measured climate parameter in measuring the at least one climate.

Reaction mixture composition module 120 is configured to compose the reaction mixture and/or process conditions according the calculation.

Reaction mixture reaction module 122 is configured to react the reaction mixture to obtain the chemical product after making the adjustment. The reaction mixture or process conditions in may adjust the reaction mixture if a calculation is made in adjustinging the reaction.

Climate parameter measuring module 124 is configured to measuring, at the production site, the at least one climate parameter used in the combined regression model before, during and/or after reacting the reaction mixture has begun.

Reaction mixture process condition adjusting module 126 is configured to adjust the reaction mixture or process conditions according to the calculation in calculating the composition.

Measurement determination module 130 is configured to determine if the measurement in measuring the at least one climate is different from a previous measurement of the at least one climate parameter.

In some implementations, at least two target physical properties are considered. In some implementations, at least two climate parameters are considered. In some implementations, the air's temperature, pressure or relative humidity or a combination thereof are considered. In some implementations, by way of non-limiting example, the chemical product is a polyurethane foam and the reaction mixture includes a polyisocyanate, a polyisocyanate-reactive compound and a blowing agent. In some implementations, composing the reaction mixture includes starting from a pre-defined standard reaction mixture composition increasing or decreasing a molar ratio of isocyanate groups to isocyanate-reactive groups.

In some implementations, composing the reaction mixture includes starting from a pre-defined standard reaction mixture composition increasing or decreasing an amount of blowing agent. In some implementations, composing the reaction mixture includes starting from a pre-defined standard reaction mixture composition increasing or decreasing relative amounts of physical and chemical blowing agents with respect to each other.

In some implementations, server(s) 102, client computing platform(s) 104, and/or external resources 132 are operatively linked via one or more electronic communication links. For example, such electronic communication links is established, at least in part, via a network such as the Internet and/or other networks. It will be appreciated that this is not intended to be limiting, and that the scope of this disclosure includes implementations in which server(s) 102, client computing platform(s) 104, and/or external resources 132 is operatively linked via some other communication media.

A given client computing platform 104 includes one or more processors configured to execute computer program modules. The computer program modules is configured to enable an expert or user associated with the given client computing platform 104 to interface with system 100 and/or external resources 132, and/or provide other functionality attributed herein to client computing platform(s) 104. By way of non-limiting example, the given client computing platform 104 includes one or more of a desktop computer, a laptop computer, a handheld computer, a tablet computing platform, a NetBook, a Smartphone, a gaming console, and/or other computing platforms.

External resources 132 include sources of information outside of system 100, external entities participating with system 100, and/or other resources. In some implementations, some or all of the functionality attributed herein to external resources 132 is provided by resources included in system 100.

Server(s) 102 include electronic storage 134, one or more processors 136, and/or other components. Server(s) 102 includes communication lines, or ports to enable the exchange of information with a network and/or other computing platforms. Illustration of server(s) 102 in FIG. 1 is not intended to be limiting. Server(s) 102 include a plurality of hardware, software, and/or firmware components operating together to provide the functionality attributed herein to server(s) 102. For example, server(s) 102 are implemented by a cloud of computing platforms operating together as server(s) 102.

Electronic storage 134 may comprise non-transitory storage media that electronically stores information. The electronic storage media of electronic storage 134 includes one or both of system storage that is provided integrally (i.e., substantially non-removable) with server(s) 102 and/or removable storage that is removably connectable to server(s) 102 via, for example, a port (e.g., a USB port, a firewire port, etc.) or a drive (e.g., a disk drive, etc.). Electronic storage 134 includes one or more of optically readable storage media (e.g., optical disks, etc.), magnetically readable storage media (e.g., magnetic tape, magnetic hard drive, floppy drive, etc.), electrical charge-based storage media (e.g., EEPROM, RAM, etc.), solid-state storage media (e.g., flash drive, etc.), and/or other electronically readable storage media. Electronic storage 134 includes one or more virtual storage resources (e.g., cloud storage, a virtual private network, and/or other virtual storage resources). Electronic storage 134 may store software algorithms, information determined by processor(s) 136, information received from server(s) 102, information received from client computing platform(s) 104, and/or other information that enables server(s) 102 to function as described herein.

Processor(s) 136 is configured to provide information processing capabilities in server(s) 102. As such, processor(s) 136 includes one or more of a digital processor, an analog processor, a digital circuit designed to process information, an analog circuit designed to process information, a state machine, and/or other mechanisms for electronically processing information. Although processor(s) 136 is shown in FIG. 1 as a single entity, this is for illustrative purposes only. In some implementations, processor(s) 136 includes a plurality of processing units. These processing units is physically located within the same device, or processor(s) 136 may represent processing functionality of a plurality of devices operating in coordination. Processor(s) 136 is configured to execute modules 108, 110, 112, 114, 118, 120, 124, 126 and/or 130, and/or other modules by software, hardware, firmware, some combination of software, hardware, and/or firmware; and/or other mechanisms for configuring processing capabilities on processor(s) 136. As used herein, the term "module" may refer to any component or set of components that perform the functionality attributed to the module. This includes one or more physical processors during execution of processor readable instructions, the processor readable instructions, circuitry, hardware, storage media, or any other components.

It should be appreciated that although modules 108, 110, 112, 114, 118, 120, 124, 126 and/or 130 are illustrated in FIG. 1 as being implemented within a single processing unit, in implementations in which processor(s) 136 includes multiple processing units, one or more of modules 108, 110, 112, 114, 118, 120, 124, 126 and/or 130 is implemented remotely from the other modules. The description of the functionality provided by the different modules 108, 110, 112, 114, 118, 120, 124, 126 and/or 130 described below is for illustrative purposes, and is not intended to be limiting, as any of modules 108, 110, 112, 114, 118, 120, 124, 126 and/or 130 may provide more or less functionality than is described. For example, one or more of modules 108, 110, 112, 114, 118, 120, 124, 126 and/or 130 is eliminated, and some or all of its functionality is provided by other ones of modules 108, 110, 112, 114, 118, 120, 124, 126 and/or 130. As another example, processor(s) 136 is configured to execute one or more additional modules that may perform some or all of the functionality attributed below to one of modules 108, 110, 112, 114, 118, 120, 124, 126 and/or 130.

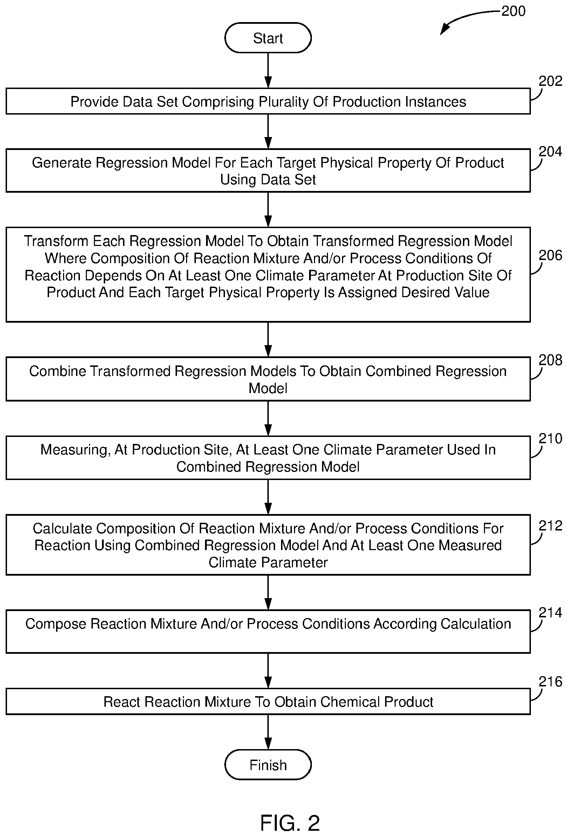

FIG. 2 illustrates a method 200 for producing a chemical product from a reaction mixture containing at least two components, in accordance with one or more implementations. The operations of method 200 presented below are intended to be illustrative. In some implementations, method 200 is accomplished with one or more additional operations not described, and/or without one or more of the operations discussed.

In some implementations, method 200 is implemented in one or more processing devices (e.g., a digital processor, an analog processor, a digital circuit designed to process information, an analog circuit designed to process information, a state machine, and/or other mechanisms for electronically processing information). The one or more processing devices includes one or more devices executing some or all of the operations of method 200 in response to instructions stored electronically on an electronic storage medium. The one or more processing devices includes one or more devices configured through hardware, firmware, and/or software to be specifically designed for execution of one or more of the operations of method 200.

An operation 202 includes providing a data set including a plurality of production instances. Each production instance includes data on the composition of the reaction mixture, at least one climate parameter at the production site of the product, and at least one target physical property of the product. Operation 202 is performed by one or more hardware processors configured by machine-readable instructions including a module that is the same as or similar to data providing module 108, in accordance with one or more implementations.

An operation 204 includes generating a regression model for each target physical property of the product using the data set. In the regression model a respective target physical property is the dependent variable and other data in the data set are independent variables. Operation 204 is performed by one or more hardware processors configured by machine-readable instructions including a module that is the same as or similar to regression model generating module 110, in accordance with one or more implementations.

An operation 206 includes transforming each regression model to obtain a transformed regression model where the composition of the reaction mixture and/or the process conditions of a reaction depends on at least one climate parameter at the production site of the product and each target physical property is assigned a desired value. Operation 206 is performed by one or more hardware processors configured by machine-readable instructions including a module that is the same as or similar to regression model transformation module 112, in accordance with one or more implementations.

An operation 208 includes combining the transformed regression models to obtain a combined regression model. Operation 208 is performed by one or more hardware processors configured by machine-readable instructions including a module that is the same as or similar to regression model combining module 114, in accordance with one or more implementations.

An operation 210 includes measuring, at the production site, at least one climate parameter used in the combined regression model. Operation 210 is performed by one or more hardware processors configured by machine-readable instructions including a module that is the same as or similar to Measuring module 116, in accordance with one or more implementations.

An operation 212 includes calculating the composition of the reaction mixture and/or the process conditions for the reaction using the combined regression model and the at least one measured climate parameter. Operation 212 is performed by one or more hardware processors configured by machine-readable instructions including a module that is the same as or similar to composition calculation module 118, in accordance with one or more implementations.

An operation 214 includes composing the reaction mixture and/or process conditions according the calculation. Operation 214 is performed by one or more hardware processors configured by machine-readable instructions including a module that is the same as or similar to reaction mixture composition module 120, in accordance with one or more implementations.

An operation 216 includes reacting the reaction mixture to obtain the chemical product. Operation 216 is performed by one or more hardware processors configured by machine-readable instructions including a module that is the same as or similar to reaction mixture reaction module 122, in accordance with one or more implementations.

The present invention will be further described in the following section without wishing to be limited by the proceedings outlined therein. A dataset having the structure given below may be used. Each row represents data for one production instance of polyurethane foam. The foam may be produced by reacting a polyol component with a polyisocyanate component. The data may be normalized as standard scores ($z = (x-\mu)/\sigma$).

Water: amount of water [g] added to the polyol component as a chemical blowing agent; ISO: NCO index of the reaction mixture; AbsHum_Mean: mean value for the absolute air humidity [g/m$^3$] at the production site; p: air pressure [kPa] at the production site; RD: raw density of the polyurethane foam [kg/m$^3$]; CH40: compression load deflection of the polyurethane foam at 40% compression [kPa].

| ISO (X1) | Water (X2) | AbsHum_Mean (X3) | P (X4) | RD | CH40 |
|---|---|---|---|---|---|
| First entry | First entry | First entry | First entry | First entry | First entry |
| ... | ... | ... | ... | ... | ... |
| Last entry | Last entry | Last entry | Last entry | Last entry | Last entry |

In alignment with the method according to the invention each row of the table represents a production instance. The composition of the reaction mixture, as it pertains to the method according to the invention, is documented in the columns "ISO" and "Water". Likewise, climate parameters are "AbsHum_Mean" and "p" and target physical properties are "RD" and "CH40".

For the raw density "RD" the following regression model may be generated:

$$
\begin{aligned}
RD = \quad & constant\_01 \\
& + constant\_02 \cdot X1 \\
& + constant\_03 \cdot X2 \\
& + constant\_04 \cdot X4
\end{aligned}
$$

In accordance with the method according to the invention the raw density is the dependent variable and a part of the other data - ISO, water and air pressure - are the independent variables. The absolute humidity has not been taken into account. Furthermore, also in accordance with the method according to the invention, the compression load deflection as the other target physical property has been excluded.

For the compression hardness "CH40" the following regression model was generated:

$$
\begin{aligned}
CH40 = \quad & constant\_05 \\
& + constant\_06 \cdot X1 \\
& + constant\_07 \cdot X2 \\
& + constant\_08 \cdot X3 \\
& + constant\_09 \cdot X4 \\
& + constant\_10 \cdot X1^2 \\
& + constant\_11 \cdot X2^2 \\
& + constant\_12 \cdot X1 \cdot X2
\end{aligned}
$$

In accordance with the method according to the invention the compression load deflection is the dependent variable and the other data - ISO, water, absolute humidity and air pressure - are the independent variables. Furthermore, also in accordance with the method according to the invention, the raw density as the other target physical property has been excluded.

The regression models were transformed as follows. The subscript "_nr" denotes "new recipe", i.e relating to the recipe for the polyurethane foam after taking climate corrections (absolute humidity and air pressure) into account. The constants constant_01 to constant_33 may be positive or negative and have small values, usually from -2 to 2.

RD - RD_nr = 0; in expanded form:

$$
\begin{aligned}
&(\text{constant\_01} \\
&+ \text{constant\_02} \cdot \text{X1} \\
&+ \text{constant\_03} \cdot \text{X2} \\
&+ \text{constant\_04} \cdot \text{X4}) \\
&- (\text{constant\_01} \\
&+ \text{constant\_02} \cdot \text{X1\_nr} \\
&+ \text{constant\_03} \cdot \text{X2\_nr} \\
&+ \text{constant\_04} \cdot \text{X4\_nr}) \\
&= 0
\end{aligned}
$$

where RD_nr has the new climate-corrected components X1_nr, X2_nr and X4_nr.
CH40-CH40_nr=0; in expanded form:

$$
\begin{aligned}
&(\text{constant\_05} \\
&+ \text{constant\_06} \cdot \text{X1} \\
&+ \text{constant\_07} \cdot \text{X2} \\
&+ \text{constant\_08} \cdot \text{X3} \\
&+ \text{constant\_09} \cdot \text{X4} \\
&+ \text{constant\_10} \cdot \text{X1}^2 \\
&+ \text{constant\_11} \cdot \text{X2}^2 \\
&+ \text{constant\_12} \cdot \text{X1} \cdot \text{X2}) \\
&- (\text{constant\_05} \\
&+ \text{constant\_06} \cdot \text{X1\_nr} \\
&+ \text{constant\_07} \cdot \text{X2\_nr} \\
&+ \text{constant\_08} \cdot \text{X3\_nr} \\
&+ \text{constant\_09} \cdot \text{X4\_nr} \\
&+ \text{constant\_10} \cdot \text{X1\_nr}^2 \\
&+ \text{constant\_11} \cdot \text{X2\_nr}^2 \\
&+ \text{constant\_12} \cdot \text{X1\_nr} \cdot \text{X2\_} \\
&= 0
\end{aligned}
$$

where CH40_nr has also the new climate-corrected components X1_nr, X2_nr and X4_nr.
The transformed regression models were combined as follows (sqrt: quare root):

$$
\begin{aligned}
\text{X1\_nr} =\ & \text{constant\_13} \\
& + \text{constant\_14} \cdot \text{X2} \\
& + \text{constant\_14} \cdot \text{X4\_nr} \\
& + \text{constant\_15} \cdot \text{X1} \\
& - \text{constant\_14} \cdot \text{X4} \\
& + \text{constant\_16} \cdot \text{sqrt}( \\
& \qquad \text{constant\_17} \\
& \qquad + \text{constant\_18} \cdot \text{X4\_nr} \\
& \qquad + \text{constant\_19} \cdot \text{X2} \\
& \qquad + \text{constant\_20} \cdot \text{X3\_nr} \\
& \qquad - \text{constant\_18} \cdot \text{X4} \\
& \qquad - \text{constant\_21} \cdot \text{X1} \\
& \qquad - \text{constant\_20} \cdot \text{X3} \\
& \qquad + \text{constant\_22} \cdot \text{X2} \cdot \text{X4\_nr} \\
& \qquad + \text{constant\_23} \cdot \text{X1} \cdot \text{X4\_nr} \\
& \qquad - \text{constant\_22} \cdot \text{X2} \cdot \text{X4} \\
& \qquad - \text{constant\_23} \cdot \text{X1} \cdot \text{X4} \\[1em]
& - \text{constant\_24} \cdot \text{X4} \cdot \text{X4\_nr} \\
& - \text{constant\_25} \cdot \text{X1} \cdot \text{X2} \\
& + \text{constant\_26} \cdot \text{X1}^2 \\
& + \text{constant\_27} \cdot \text{X4}^2 \\
& + \text{constant\_27} \cdot \text{X4\_nr}^2 \\
& + \text{constant\_28} \cdot \text{X2}^2 )
\end{aligned}
$$

$$X2\_nr = -\, constant\_29$$
$$+\, constant\_30 \cdot X2$$
$$+\, constant\_31 \cdot X1$$
$$+\, constant\_32 \cdot X4\_nr$$
$$-\, constant\_32 \cdot X4$$
$$-\, constant\_33 \cdot sqrt($$
$$constant\_17$$
$$+\, constant\_18 \cdot X4\_nr$$
$$+\, constant\_19 \cdot X2$$
$$+\, constant\_20 \cdot X3\_nr$$
$$-\, constant\_18 \cdot X4$$
$$-\, constant\_21 \cdot X1$$
$$-\, constant\_20 \cdot X3$$
$$+\, constant\_22 \cdot X2 \cdot X4\_nr$$
$$+\, constant\_23 \cdot X1 \cdot X4\_nr$$
$$-\, constant\_22 \cdot X2 \cdot X4$$
$$-\, constant\_23 \cdot X1 \cdot X4$$
$$-\, constant\_24 \cdot X4 \cdot X4\_nr$$
$$-\, constant\_25 \cdot X1 \cdot X2$$
$$+\, constant\_26 \cdot X1^2$$
$$+\, constant\_27 \cdot X4^2$$
$$+\, constant\_27 \cdot X4\_nr^2$$
$$+\, constant\_28 \cdot X2^2)$$

X3_nr and X4_nr are the new measured absolute humidity and air pressure values. X3 and X4 are the standard lab climate conditions.

**Claims**

1. A method of producing a chemical product from a reaction mixture containing at least two components, comprising:

   I) providing a data set comprising a plurality of production instances, each production instance comprising data on (a) the composition of the reaction mixture and/or process conditions, (b) at least one climate parameter at the production site of the product, and (c) at least one target physical property of the product;
   II) generating a regression model for each target physical property of the product using the data set, wherein in the regression model a respective target physical property is the dependent variable and at least part of the other data in the data set, excluding any remaining target physical properties, are independent variables;
   III) transforming each regression model to obtain a transformed regression model where a) the composition of the reaction mixture and/or b) the process conditions of a reaction depends on at least one climate parameter at the production site of the product and each target physical property is assigned a desired value;
   IV) combining the transformed regression models to obtain a combined regression model;
   V) measuring, at the production site, the at least one climate parameter used in the combined regression model;
   VI) calculating a) the composition of the reaction mixture and/or b) the process conditions for the reaction, using the combined regression model and the at least one measured climate parameter;
   VII) composing the reaction mixture and/or process conditions according the calculation; and

VIII) reacting the reaction mixture to obtain the chemical product.

2. The method of claim 1, wherein the data set is processed prior to step I) using a statistical method to remove outliers.

3. The method of claim 1, wherein step II) comprises:

splitting the data set into a training data set and a testing data set,
generating each regression model using the training data set, and
validating the regression model against the testing data set.

4. The method of claim 3, wherein the data set is grouped into clusters and each cluster is split into a training data set and a testing data set for generating the regression models.

5. The method of claim 1, wherein the at least one climate parameter at the production site is the air pressure, the air temperature, the air's relative humidity or a combination thereof.

6. The method of claim 1, wherein the at least one target physical property is the raw density according to DIN EN ISO 845 and/or the compression load deflection at 40% compression according to EN ISO 3386.

7. The method of claim 1, wherein the chemical product is a polyurethane foam and the reaction mixture comprises a polyisocyanate, a polyisocyanate-reactive compound and a blowing agent.

8. The method of claim 1, wherein composing the reaction mixture in step VII) comprises, starting from a pre-defined standard reaction mixture composition:

(a) increasing or decreasing a molar ratio of isocyanate groups to isocyanate-reactive groups;
(b) increasing or decreasing an amount of blowing agent;
(c) increasing or decreasing relative amounts of physical and chemical blowing agents with respect to each other; or
(d) a combination thereof.

9. The method of claim 1, further comprising:

IX) measuring, at the production site, the at least one climate parameter used in the combined regression model after step VIII has begun;
X) calculating the composition of the reaction mixture or process conditions of the reaction using the combined regression model and the at least one measured climate parameter in step IX;
XI) adjusting, or deciding not to adjust, the reaction mixture or process conditions according to the calculation in step X; and
XII) reacting the reaction mixture to obtain the chemical product after making the adjustment or the decision not to adjust, the reaction mixture or process conditions in step XI.

10. The method of Claim 9, wherein steps IX to XII are repeated at least once every 60 seconds, at least once every 30 seconds, or continuously.

11. The method of claim 1, further comprising:

IX') measuring, at the production site, the at least one climate parameter used in the combined regression model after step VIII has begun;
X') determining if the measurement in step IX' is different from a previous measurement of the at least one climate parameter;
XI') if the measurement has been determined to be different, then calculating the composition of the reaction mixture or process conditions of the reaction using the combined regression model and the at least one measured climate parameter in step IX';
XII') adjusting the reaction mixture or process conditions according to the calculation in step XI', if such a calculation is made; and
XIII') reacting the reaction mixture to obtain the chemical product after making the adjustment, the reaction mixture or process conditions in step XII', if a calculation is made in step XI.

**12.** The method of Claim 11, wherein steps IX' to XI' are repeated at least once every 60 seconds, at least once every 30 seconds, or continuously.

**13.** A system configured for producing a chemical product from a reaction mixture containing at least two components, the system comprising:

one or more hardware processors configured by machine-readable instructions to:

provide a data set comprising a plurality of production instances, each production instance comprising data on the composition of the reaction mixture, at least one climate parameter at the production site of the product, and at least one target physical property of the product;

generate a regression model for each target physical property of the product using the data set, wherein in the regression model a respective target physical property is the dependent variable and other data in the data set are independent variables;

transform each regression model to obtain a transformed regression model where the composition of the reaction mixture and/or the process conditions of a reaction depends on at least one climate parameter at the production site of the product and each target physical property is assigned a desired value;

combine the transformed regression models to obtain a combined regression model;

measuring, at the production site, at least one climate parameter used in the combined regression model;

calculate the composition of the reaction mixture and/or the process conditions for the reaction using the combined regression model and the at least one measured climate parameter; and

compose the reaction mixture and/or process conditions according the calculation;

and a reactor for reacting the reaction mixture to obtain the chemical product.

**14.** A computing platform configured for producing a chemical product from a reaction mixture containing at least two components, the computing platform comprising:

a non-transient computer-readable storage medium having executable instructions embodied thereon; and

one or more hardware processors configured to execute the instructions to:

provide a data set comprising a plurality of production instances, each production instance comprising data on the composition of the reaction mixture, at least one climate parameter at the production site of the product, and at least one target physical property of the product;

generate a regression model for each target physical property of the product using the data set, wherein in the regression model a respective target physical property is the dependent variable and other data in the data set are independent variables;

transform each regression model to obtain a transformed regression model where the composition of the reaction mixture and/or the process conditions of a reaction depends on at least one climate parameter at the production site of the product and each target physical property is assigned a desired value;

combine the transformed regression models to obtain a combined regression model;

measuring, at the production site, at least one climate parameter used in the combined regression model;

calculate the composition of the reaction mixture and/or the process conditions for the reaction using the combined regression model and the at least one measured climate parameter; and

compose the reaction mixture and/or process conditions according the calculation.

**15.** The platform according to claim 14, wherein the platform is in data communication with a reactor for reacting the reaction mixture to obtain the chemical product.

**Patentansprüche**

**1.** Verfahren zur Herstellung eines chemischen Produkts aus einer Reaktionsmischung, die mindestens zwei Komponenten enthält, Folgendes umfassend:

I) Bereitstellen eines Datensatzes, der mehrere Herstellungsinstanzen umfasst, wobei jede Herstellungsinstanz Daten über (a) die Zusammensetzung der Reaktionsmischung und/oder die Prozessbedingungen, (b) mindestens einen Klimaparameter am Herstellungsort des Produkts und (c) mindestens eine physikalische Zieleigenschaft des Produkts umfasst;

II) Erzeugen eines Regressionsmodells für jede physikalische Zieleigenschaft des Produkts unter Verwendung des Datensatzes, wobei in dem Regressionsmodell eine jeweilige physikalische Zieleigenschaft die abhängige Variable ist und mindestens ein Teil der anderen Daten in dem Datensatz, außer allen verbleibenden physikalischen Zieleigenschaften, unabhängige Variablen sind;

III) Transformieren jedes Regressionsmodells, um ein transformiertes Regressionsmodell zu erhalten, bei dem a) die Zusammensetzung der Reaktionsmischung und/oder b) die Prozessbedingungen einer Reaktion von mindestens einem Klimaparameter an dem Herstellungsort des Produkts abhängt und jeder physikalischen Zieleigenschaft ein erwünschter Wert zugewiesen wird;

IV) Kombinieren der transformierten Regressionsmodelle, um ein kombiniertes Regressionsmodell zu erhalten;

V) Messen, an dem Herstellungsort, des mindestens einen Klimaparameters, der in dem kombinierten Regressionsmodell verwendet wird;

VI) Berechnen a) der Zusammensetzung der Reaktionsmischung und/oder b) der Prozessbedingungen für die Reaktion unter Verwendung des kombinierten Regressionsmodells und des mindestens einen gemessenen Klimaparameters;

VII) Zusammensetzen der Reaktionsmischung und/oder der Prozessbedingungen gemäß der Berechnung; und

VIII) Reagieren der Reaktionsmischung, um das chemische Produkt zu erhalten.

2. Verfahren nach Anspruch 1, wobei der Datensatz vor dem Schritt I) unter Verwendung eines statistischen Verfahrens verarbeitet wird, um Ausreißer zu entfernen.

3. Verfahren nach Anspruch 1, wobei Schritt II) Folgendes umfasst:

Trennen des Datensatzes in einen Trainingsdatensatz und einen Prüfdatensatz,
Erzeugen jedes Regressionsmodells unter Verwendung des Trainingsdatensatzes und
Validieren des Regressionsmodells gegen den Prüfdatensatz.

4. Verfahren nach Anspruch 3, wobei der Datensatz in Cluster gruppiert wird und jeder Cluster in einen Trainingsdatensatz und einen Prüfdatensatz zum Erzeugen der Regressionsmodelle geteilt wird.

5. Verfahren nach Anspruch 1, wobei der mindestens eine Klimaparameter an dem Herstellungsort der Luftdruck, die Lufttemperatur, die relative Luftfeuchtigkeit der Luft oder eine Kombination davon ist.

6. Verfahren nach Anspruch 1, wobei die mindestens eine physikalische Zieleigenschaft die Rohdichte gemäß DIN EN ISO 845 und/oder die Kompressionslastdurchbiegung bei 40 % Kompression gemäß EN ISO 3386 ist.

7. Verfahren nach Anspruch 1, wobei das chemische Produkt ein Polyurethan-Schaum ist und die Reaktionsmischung ein Polyisocyanat, eine mit Polyisocyanat reaktionsfähige Verbindung und ein Blähmittel enthält.

8. Verfahren nach Anspruch 1, wobei Zusammensetzen der Reaktionsmischung in Schritt VII), angefangen von einer vordefinierten Normzusammensetzung der Reaktionsmischung, Folgendes umfasst:

(a) Erhöhen oder Vermindern eines molaren Verhältnisses von Isocyanat-Gruppen zu mit Isocyanat reaktionsfähigen Gruppen;
(b) Erhöhen oder Vermindern einer Menge Blähmittel;
(c) Erhöhen oder Vermindern relativer Mengen physikalischer und chemischer Blähmittel zueinander; oder
(d) eine Kombination davon.

9. Verfahren nach Anspruch 1, weiterhin Folgendes umfassend:

IX) Messen, an dem Herstellungsort, des mindestens einen Klimaparameters, der in dem kombinierten Regressionsmodell verwendet wird, nachdem der Schritt VIII begonnen hat;

X) Berechnen der Zusammensetzung der Reaktionsmischung oder der Prozessbedingungen der Reaktion unter Verwendung des kombinierten Regressionsmodells und des mindestens einen in Schritt IX gemessenen Klimaparameters;

XI) Einstellen oder Entscheiden, diese nicht einzustellen, der Reaktionsmischung oder der Prozessbedingungen gemäß der Berechnung in Schritt X; und

XII) Reagieren der Reaktionsmischung, um das chemische Produkt zu erhalten, nachdem die Einstellung oder die Entscheidung, nicht einzustellen, der Reaktionsmischung oder der Prozessbedingungen in Schritt XI vorgenommen wurde.

10. Verfahren nach Anspruch 9, wobei die Schritte IX bis XII mindestens einmal alle 60 Sekunden, mindestens einmal alle 30 Sekunden oder kontinuierlich wiederholt werden.

11. Verfahren nach Anspruch 1, weiterhin Folgendes umfassend:

IX') Messen, an dem Herstellungsort, des mindestens einen Klimaparameters, der in dem kombinierten Regressionsmodell verwendet wird, nachdem der Schritt VIII begonnen hat;
X') Bestimmen, wenn die Messung in Schritt IX' von einer vorhergehenden Messung des mindestens einen Klimaparameters verschieden ist;
XI') wenn bestimmt wird, dass die Messung verschieden ist, dann Berechnen der Zusammensetzung der Reaktionsmischung oder der Prozessbedingungen der Reaktion unter Verwendung des kombinierten Regressionsmodells und des mindestens einen in Schritt IX' gemessenen Klimaparameters;
XII') Einstellen der Reaktionsmischung oder der Prozessbedingungen gemäß der Berechnung in Schritt XI', wenn eine derartige Berechnung vorgenommen wird; und
XIII') Reagieren der Reaktionsmischung, um das chemische Produkt zu erhalten, nach Vornehmen der Einstellung der Reaktionsmischung oder der Prozessbedingungen in Schritt XII', wenn eine Berechnung in Schritt XI vorgenommen wurde.

12. Verfahren nach Anspruch 11, wobei die Schritte IX' bis XI' mindestens einmal alle 60 Sekunden, mindestens einmal alle 30 Sekunden oder kontinuierlich wiederholt werden.

13. System, das zum Herstellen eines chemischen Produkts aus einer Reaktionsmischung eingerichtet ist, die mindestens zwei Komponenten enthält, das System Folgendes umfassend:

einen oder mehrere Hardware-Prozessoren, die durch maschinenlesbare Befehle eingerichtet sind, um:

einen Datensatz bereitzustellen, der mehrere Herstellungsinstanzen umfasst, wobei jede Herstellungsinstanz Daten über die Zusammensetzung der Reaktionsmischung, mindestens einen Klimaparameter am Herstellungsort des Produkts und mindestens eine physikalische Zieleigenschaft des Produkts umfasst;
ein Regressionsmodell für jede physikalische Zieleigenschaft des Produkts unter Verwendung des Datensatzes zu erzeugen, wobei in dem Regressionsmodell eine jeweilige physikalische Zieleigenschaft die abhängige Variable ist und andere Daten in dem Datensatz unabhängige Variablen sind;
jedes Regressionsmodell zu transformieren, um ein transformiertes Regressionsmodell zu erhalten, bei dem die Zusammensetzung der Reaktionsmischung und/oder die Prozessbedingungen einer Reaktion von mindestens einem Klimaparameter an dem Herstellungsort des Produkts abhängt und jeder physikalischen Zieleigenschaft ein erwünschter Wert zugewiesen wird;
die transformierten Regressionsmodelle zu kombinieren, um ein kombiniertes Regressionsmodell zu erhalten;
an dem Herstellungsort mindestens einen Klimaparameter zu messen, der in dem kombinierten Regressionsmodell verwendet wird;
die Zusammensetzung der Reaktionsmischung und/oder die Prozessbedingungen für die Reaktion unter Verwendung des kombinierten Regressionsmodells und des mindestens einen gemessenen Klimaparameters zu berechnen; und
die Reaktionsmischung und/oder die Prozessbedingungen gemäß der Berechnung zusammenzusetzen;

und einen Reaktor zum Reagieren der Reaktionsmischung, um das chemische Produkt zu erhalten.

14. Rechnerplattform, die zum Herstellen eines chemischen Produkts aus einer Reaktionsmischung eingerichtet ist, die mindestens zwei Komponenten enthält, die Rechnerplattform Folgendes umfassend:

ein nichtflüchtiges computerlesbares Speichermedium mit darauf enthaltenen ausführbaren Befehlen; und
einen oder mehrere Hardware-Prozessoren, die eingerichtet sind, um die Befehle auszuführen, um:

einen Datensatz bereitzustellen, der mehrere Herstellungsinstanzen umfasst, wobei jede Herstellungsinstanz Daten über die Zusammensetzung der Reaktionsmischung, mindestens einen Klimaparameter am Herstellungsort des Produkts und mindestens eine physikalische Zieleigenschaft des Produkts umfasst; ein Regressionsmodell für jede physikalische Zieleigenschaft des Produkts unter Verwendung des Datensatzes zu erzeugen, wobei in dem Regressionsmodell eine jeweilige physikalische Zieleigenschaft die abhängige Variable ist und andere Daten in dem Datensatz unabhängige Variablen sind; jedes Regressionsmodell zu transformieren, um ein transformiertes Regressionsmodell zu erhalten, bei dem die Zusammensetzung der Reaktionsmischung und/oder die Prozessbedingungen einer Reaktion von mindestens einem Klimaparameter an dem Herstellungsort des Produkts abhängt und jeder physikalischen Zieleigenschaft ein erwünschter Wert zugewiesen wird; die transformierten Regressionsmodelle zu kombinieren, um ein kombiniertes Regressionsmodell zu erhalten; an dem Herstellungsort mindestens einen Klimaparameter zu messen, der in dem kombinierten Regressionsmodell verwendet wird; die Zusammensetzung der Reaktionsmischung und/oder die Prozessbedingungen für die Reaktion unter Verwendung des kombinierten Regressionsmodells und des mindestens einen gemessenen Klimaparameters zu berechnen; und die Reaktionsmischung und/oder die Prozessbedingungen gemäß der Berechnung zusammenzusetzen.

15. Plattform nach Anspruch 14, wobei die Plattform in Datenkommunikation mit einem Reaktor zum Reagieren der Reaktionsmischung steht, um das chemische Produkt zu erhalten.

## Revendications

1. Procédé de production d'un produit chimique à partir d'un mélange de réaction contenant au moins deux composants, comprenant :

    I) la fourniture d'un jeu de données comprenant une pluralité de cas de production, chaque cas de production comprenant des données sur (a) la composition du mélange de réaction et/ou des conditions de traitement, (b) au moins un paramètre climatique au site de production du produit, et (c) au moins une propriété physique cible du produit ;
    II) la génération d'un modèle de régression pour chaque propriété physique cible du produit en utilisant le jeu de données, dans lequel, dans le modèle de régression, une propriété physique cible respective est la variable dépendante et au moins une partie des autres données dans le jeu de données, à l'exclusion de toutes autres propriétés physiques cibles, est composée de variables indépendantes ;
    III) la transformation de chaque modèle de régression pour obtenir un modèle de régression transformé où a) la composition du mélange de réaction et/ou b) des conditions de traitement d'une réaction dépend d'au moins un paramètre climatique au site de production du produit, et une valeur souhaitée est attribuée à chaque propriété physique cible ;
    IV) la combinaison des modèles de régression transformés pour obtenir un modèle de régression combiné ;
    V) la mesure, au site de production, de l'au moins un paramètre climatique utilisé dans le modèle de régression combiné ;
    VI) le calcul a) de la composition du mélange de réaction et/ou b) des conditions de traitement pour la réaction, en utilisant le modèle de régression combiné et l'au moins un paramètre climatique mesuré ;
    VII) la composition du mélange de réaction et/ou des conditions de traitement selon le calcul ; et
    VIII) la mise en réaction du mélange de réaction pour obtenir le produit chimique.

2. Procédé selon la revendication 1, dans lequel le jeu de données est traité avant l'étape I) en utilisant un procédé statistique pour éliminer des aberrances.

3. Procédé selon la revendication 1, dans lequel l'étape II) comprend :

    la division du jeu de données en un jeu de données d'apprentissage et un jeu de données d'essai,
    la génération de chaque modèle de régression en utilisant le jeu de données d'apprentissage, et
    la validation du modèle de régression en comparaison au jeu de données d'essai.

4. Procédé selon la revendication 3, dans lequel le jeu de données est groupé en grappes et chaque grappe est divisée en un jeu de données d'apprentissage et un jeu de données d'essai pour générer les modèles de régression.

**5.** Procédé selon la revendication 1, dans lequel l'au moins un paramètre climatique au site de production est la pression de l'air, la température de l'air, l'humidité relative de l'air ou une association de celles-ci.

**6.** Procédé selon la revendication 1, dans lequel l'au moins une propriété physique cible est la densité brute selon DIN EN ISO 845 et/ou la déflexion de charge de compression à compression de 40 % selon EN ISO 3386.

**7.** Procédé selon la revendication 1, dans lequel le produit chimique est une mousse polyuréthane et le mélange de réaction comprend un polyisocyanate, un composé réactif avec les polyisocyanates et un agent gonflant.

**8.** Procédé selon la revendication 1, dans lequel la composition du mélange de réaction dans l'étape VII) comprend, en commençant à partir d'une composition de mélange de réaction standard prédéfinie :

(a) l'augmentation ou la réduction d'un rapport molaire de groupes isocyanate par rapport à des groupes réactifs avec les isocyanates ;
(b) l'augmentation ou la réduction d'une quantité d'agent gonflant ;
(c) l'augmentation ou la réduction de quantités relatives d'agents gonflants physiques et chimiques les uns par rapport aux autres ; ou
(d) une association de celles-ci.

**9.** Procédé selon la revendication 1, comprenant en outre :

IX) la mesure, au site de production, de l'au moins un paramètre climatique utilisé dans le modèle de régression combiné après que l'étape VIII a commencé ;
X) le calcul de la composition du mélange de réaction ou des conditions de traitement de la réaction en utilisant le modèle de régression combiné et l'au moins un paramètre climatique mesuré dans l'étape IX ;
XI) l'ajustement, ou la décision de ne pas réaliser d'ajustement, du mélange de réaction ou des conditions de traitement selon le calcul dans l'étape X ; et
XII) la mise en réaction du mélange de réaction pour obtenir le produit chimique après la réalisation de l'ajustement, ou la décision de ne pas réaliser l'ajustement, du mélange de réaction ou des conditions de traitement dans l'étape XI.

**10.** Procédé selon la revendication 9, dans lequel les étapes IX à XII sont répétées au moins une fois toutes les 60 secondes, au moins une fois toutes les 30 secondes, ou en continu.

**11.** Procédé selon la revendication 1, comprenant en outre :

IX') la mesure, au site de production, de l'au moins un paramètre climatique utilisé dans le modèle de régression combiné après que l'étape VIII a commencé ;
X') la détermination que la mesure dans l'étape IX' est, ou n'est pas, différente d'une mesure précédente de l'au moins un paramètre climatique ;
XI') s'il a été déterminé que la mesure est différente, alors le calcul de la composition du mélange de réaction ou des conditions de traitement de la réaction en utilisant le modèle de régression combiné et l'au moins un paramètre climatique mesuré dans l'étape IX' ;
XII') l'ajustement du mélange de réaction ou des conditions de traitement selon le calcul dans l'étape XI', si un tel calcul est réalisé ; et
XIII') la mise en réaction du mélange de réaction pour obtenir le produit chimique après la réalisation de l'ajustement du mélange de réaction ou des conditions de traitement dans l'étape XII', si un calcul est réalisé dans l'étape XI.

**12.** Procédé selon la revendication 11, dans lequel les étapes IX' à XI' sont répétées au moins une fois toutes les 60 secondes, au moins une fois toutes les 30 secondes, ou en continu.

**13.** Système configuré pour produire un produit chimique à partir d'un mélange de réaction contenant au moins deux composants, le système comprenant :

un ou plusieurs processeurs matériels configurés par des instructions lisibles par machine pour :

fournir un jeu de données comprenant une pluralité de cas de production, chaque cas de production comprenant des données sur la composition du mélange de réaction, au moins un paramètre climatique au site de production du produit, et au moins une propriété physique cible du produit ;

générer un modèle de régression pour chaque propriété physique cible du produit en utilisant le jeu de données, dans lequel, dans le modèle de régression, une propriété physique cible respective est la variable dépendante et d'autres données dans le jeu de données sont des variables indépendantes ;

transformer chaque modèle de régression pour obtenir un modèle de régression transformé où la composition du mélange de réaction et/ou de conditions de traitement d'une réaction dépend d'au moins un paramètre climatique au site de production du produit, et une valeur souhaitée est attribuée à chaque propriété physique cible ;

combiner les modèles de régression transformés pour obtenir un modèle de régression combiné ;

mesurer, au site de production, au moins un paramètre climatique utilisé dans le modèle de régression combiné ;

calculer la composition du mélange de réaction et/ou des conditions de traitement pour la réaction en utilisant le modèle de régression combiné et l'au moins un paramètre climatique mesuré ; et

composer le mélange de réaction et/ou les conditions de traitement selon le calcul ; et

un réacteur pour mettre en réaction le mélange de réaction pour obtenir le produit chimique.

14. Plate-forme informatique configurée pour produire un produit chimique à partir d'un mélange de réaction contenant au moins deux composants, la plate-forme informatique comprenant :

un support de stockage non transitoire lisible par ordinateur ayant des instructions exécutables comprises sur celui-ci ; et

un ou plusieurs processeurs matériels configurés pour exécuter des instructions pour :

fournir un jeu de données comprenant une pluralité de cas de production, chaque cas de production comprenant des données sur la composition du mélange de réaction, au moins un paramètre climatique au site de production du produit, et au moins une propriété physique cible du produit ;

générer un modèle de régression pour chaque propriété physique cible du produit en utilisant le jeu de données, dans laquelle, dans le modèle de régression, une propriété physique cible respective est la variable dépendante et d'autres données dans le jeu de données sont des variables indépendantes ;

transformer chaque modèle de régression pour obtenir un modèle de régression transformé où la composition du mélange de réaction et/ou de conditions de traitement d'une réaction dépend d'au moins un paramètre climatique au site de production du produit, et une valeur souhaitée est attribuée à chaque propriété physique cible ;

combiner les modèles de régression transformés pour obtenir un modèle de régression combiné ;

mesurer, au site de production, au moins un paramètre climatique utilisé dans le modèle de régression combiné ;

calculer la composition du mélange de réaction et/ou des conditions de traitement pour la réaction en utilisant le modèle de régression combiné et l'au moins un paramètre climatique mesuré ; et

composer le mélange de réaction et/ou les conditions de traitement selon le calcul.

15. Plate-forme selon la revendication 14, dans laquelle la plate-forme est en communication de données avec un réacteur pour mettre en réaction le mélange de réaction pour obtenir le produit chimique.

Server(s) 102

Electronic Storage 134

Processor(s) 136

Machine-Readable Instructions 106

Data Providing Module 108

Regression Model Generating Module 110

Regression Model Transformation Module 112

Regression Model Combining Module 114

Composition Calculation Module 118

Reaction Mixture Composition Module 120

Climate Parameter Measuringing Module 124

Reaction Mixture Process Condition Adjusting Module 126

Measurement Determination Module 130

Client Computing Platform(s) 104

External Resources 132

100

FIG. 1

200

Start

Provide Data Set Comprising Plurality Of Production Instances

202

Generate Regression Model For Each Target Physical Property Of Product Using Data Set

204

Transform Each Regression Model To Obtain Transformed Regression Model Where Composition Of Reaction Mixture And/or Process Conditions Of Reaction Depends On At Least One Climate Parameter At Production Site Of Product And Each Target Physical Property Is Assigned Desired Value

206

Combine Transformed Regression Models To Obtain Combined Regression Model

208

Measuring, At Production Site, At Least One Climate Parameter Used In Combined Regression Model

210

Calculate Composition Of Reaction Mixture And/or Process Conditions For Reaction Using Combined Regression Model And At Least One Measured Climate Parameter

212

Compose Reaction Mixture And/or Process Conditions According Calculation

214

React Reaction Mixture To Obtain Chemical Product

216

Finish

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005187445 A1

**Non-patent literature cited in the description**

- **R. SCHIFFAUER ; C. DEN HEIJER.** Flexible Polyurethane Slabstock Foam: The Influence of Formulation, Climatic Conditions and Storage Conditions on Foam Properties. *Journal of Cellular Plastics,* January 1983, 61-64
- **R. SCHIFFAUER.** A Comparison between Conventional Slabstock, High Resilience Slabstock and High Resilience Molded Foams. *Journal of Cellular Plastics,* 1996, vol. 32, 318-354
- **HUBERT EHBING ; KARL-HEINZ DÖRNER ; HANS-FRIEDRICH WALTER ; BOLKO RAFFEL ; R. NÄSCHER.** Innovative Produktionsoptimierung für die Blockschaum-Fertigung. *the conference proceedings "PUR 2002 - Automobil - Comfort - Struktur - Kühlen - Bauen,* October 2002, 192-144